# FASCICULE DE BREVET EUROPEEN

(11) **EP 3 665 268 B1**
(45) Date de publication et mention de la délivrance du brevet: **24.03.2021**
(21) Numéro de dépôt: 18789195.7
(22) Date de dépôt: 01.08.2018
(51) Int. Cl.: C12N 1/20

(54) **MILIEU DE CULTURE POLYVALENT POUR BACTÉRIES ANAÉROBIES EN AÉROBIE**
POLYVALENTES KULTURMEDIUM FÜR ANAEROBE BAKTERIEN IM AEROBEN BEREICH
POLYVALENT CULTURE MEDIUM FOR ANAEROBIC BACTERIA UNDER AEROBIC CONDITIONS

(30) Priorité: 08.08.2017 FR 1757574
(43) Date de publication de la demande: 17.06.2020
(73) Titulaire: Fondation Méditerranée Infection, 13005 Marseille (FR); Université d'Aix Marseille, 13284 Marseille Cedex 07 (FR); Assistance Publique - Hôpitaux de Marseille, 13005 Marseille (FR); Centre National de la Recherche Scientifique (CNRS), 75016 Paris (FR); INSERM (Institut National de la Santé et de la Recherche Médicale), 75013 Paris (FR)
(72) Inventeur: RAOULT, Didier, 13008 Marseille (FR); KHELAIFIA, Saber, 13010 Marseille (FR); BONNET, Marion, 13010 Marseille (FR)
(74) Mandataire: Cabinet Beau de Loménie
(86) Numéro de dépôt international: PCT/FR2018/051987
(87) Numéro de publication internationale: WO 2019/030446

(56) Documents cités:
- WO-A1-2014/064359
- N. DIONE ET AL: "A quasi-universal medium to break the aerobic/anaerobic bacterial culture dichotomy in clinical microbiology", CLINICAL MICROBIOLOGY AND INFECTION., vol. 22, no. 1, 1 janvier 2016 (2016-01-01), pages 53-58, XP055361668, United Kingdom, Switzerland ISSN: 1198-743X, DOI: 10.1016/j.cmi.2015.10.032 cité dans la demande
- KHELAIFIA S ET AL: "Aerobic culture of methanogenic archaea without an external source of hydrogen", EUROPEAN JOURNAL OF CLINICAL MICROBIOLOGY & INFECTIOUS DISEASES, SPRINGER, WIESBADEN, DE, vol. 35, no. 6, 24 mars 2016 (2016-03-24), pages 985-991, XP035893122, ISSN: 0934-9723, DOI: 10.1007/S10096-016-2627-7 [extrait le 2016-03-24]

## Description

La présente invention concerne un milieu de culture polyvalent pour bactéries anaérobies en aérobie dans des flacons d'hémoculture.

Les bactéries anaérobies sont des bactéries sensibles à l'oxygène. Il existe les bactéries anaérobies strictes (extrêmement sensible à l'oxygène), qui ne doivent en aucun cas être au contact de l'oxygène pour avoir une croissance, et les bactéries aéro-anaérobies tolérantes, qui peuvent être exposées à une faible concentration en oxygène, sans que leur croissance ne soit inhibée. Cette sensibilité à l'oxygène est liée au fait qu'il manque à ces bactéries des systèmes enzymatiques. Ces derniers permettent normalement la détoxification des espèces réactives de l'oxygène ; l'absence de cette détoxification conduit à une action toxique de l'oxygène sur ces bactéries. Pour permettre la croissance de ces bactéries anaérobies strictes ou aéro-anaérobies tolérantes, des procédés de culture « sans oxygène » ont été mis en place [1].

Raccourcir le délai pour le diagnostic des bactériémies reste un défi majeur pour les microbiologistes. La fiabilité et la rapidité du diagnostic sont des éléments clés pour une amélioration de la prise en charge des patients. A cet égard, malgré la commercialisation de nombreux systèmes moléculaires, la culture en milieu de culture liquide demeure à ce jour le standard optimal dans le diagnostic des bactériémies dans des échantillons cliniques de prélèvement de fluide ou secrétions corporels tel que échantillon de selles, de crachats, de secrétions vaginales, ou échantillons sanguins. Il repose sur l'incubation de deux flacons en contenant, pour bactéries anaérobie et respectivement aérobie dans des systèmes de culture automatisés détectant la croissance bactérienne.

Actuellement, le diagnostic hospitalier des bactéries anaérobies se fait le plus généralement en culture liquide dans des flacons de culture en milieu liquide contenant une atmosphère anaérobie, c'est-à-dire une atmosphère qui ne contient pas d'oxygène, où l'oxygène atmosphérique est remplacé par de l'azote, en utilisant un procédé de dégazage. Les bactéries anaérobies et aérobies sont cultivées dans des flacons de culture différents, à savoir qu'on réalise l'incubation de deux flacons, anaérobie et aérobie dans des systèmes de culture automatisés détectant la croissance bactérienne.

Afin de minimiser le nombre de flacons à prélever pour diagnostiquer une bactériémie ou pour cultiver des bactéries dans le cadre de la recherche, les inventeurs ont cherché à fournir un milieu de culture liquide qui permettrait à la fois la croissance des bactéries aérobies et des anaérobies en atmosphère aérobie.

Il a été récemment rapporté la mise en évidence de la croissance en atmosphère aérobie d'espèces de bactéries strictement anaérobies comprenant un milieu de culture de base supplémenté par des composés antioxydants [2].

Dans WO2014/064359 et WO2015/162377, on a décrit des milieux de culture enrichis en agents antioxydants à savoir l'acide urique, l'acide ascorbique, le glutathion et l'hydrosulfure de sodium qui permettent d'améliorer et faciliter les conditions de croissance en culture acellulaire des bactéries anaérobies strictes ou des bactéries dont la croissance est sensible à la tension en oxygène et notamment des bactéries qui tolèrent mal des tensions élevées d'oxygène et pour lesquelles une croissance optimale de la dite bactérie requière une atmosphère d'incubation à teneur en oxygène relativement réduite par rapport à la teneur en oxygène de l'air. Les meilleurs résultats décrits dans WO 2015/162377 concernaient des milieux de culture supplémentés en un mélange d'antioxydants incluant l'acide urique.

Après avoir testé les milieux de culture supplémentés par des composés antioxydants décrits dans WO2014/064359 et WO2015/162377 sous forme liquide pour hémoculture, les inventeurs ont remarqué que certaines bactéries anaérobies, incluant des bactéries parmi les plus communément retrouvées en pratiques hospitalières, n'avaient pas de croissance en aérobie, à savoir *Bacteroides fragilis, Bacteroides nordii, Bacteroides thetaiotaomicron, Clostridium beijeirinckii, Clostridium butyricum, Clostridium massilioamazoniensis, Clostridium irregulare, Finegoldia magna, Propionibacterium acnes et Propionibacterium avidum.*

Sur la base des mélanges de composés antioxydants décrits antérieurement, le meilleur mélange apparaissait être la combinaison de hydrosulfure de sodium (Na₂S), L-Cystéine, acide ascorbique, acide urique et glutathion. Mais, les milieux de culture pour bactéries anaérobies supplémentés par ce mélange de composés antioxydant ne permettaient pas la croissance des bactéries anaérobies difficiles à cultiver en aérobies décrites ci-dessus.

En outre, selon la présente invention, les inventeurs ont recherché un nouveau milieu de culture liquide polyvalent pour les cultures de bactéries aérobies et anaérobies en atmosphère aérobie en l'absence d'acide urique car celui-ci présente des propriétés de dilution réduite ce qui se traduit par des taux de dissolution en solution aqueuse non reproductibles de façon déterminée quantitativement d'échantillon à échantillon.

Selon la présente invention, les inventeurs ont donc recherché un nouveau milieu de culture liquide polyvalent pour les cultures de bactéries aérobies et anaérobies en atmosphère aérobie en l'absence d'acide urique et présentant en outre des propriétés améliorées de croissance notamment vis-à-vis des bactéries anaérobies fastidieuses communément rencontrées telles que *Bacteroides fragilis, Bacteroides nordii, Bacteroides thetaiotaomicron, Clostridium beijeirinckii, Clostridium butyricum, Clostridium massilioamazoniensis, Clostridium irregulare, Finegoldia magna, Propionibacterium acnes et Propionibacterium avidum.*

Pour ce faire, les inventeurs ont testé un grand nombre de combinaisons de divers composés antioxydants rapportés ci-après (*cf* tableau 2) qui ont permis de définir de manière surprenante un milieu de culture liquide supplémenté en composés antioxydants présentant des propriétés de croissance de bactéries anaérobies en aérobie améliorées par rapport à la combinaison de hydrosulfure de sodium (Na₂S), L-Cystéine, acide ascorbique et glutathion avec ou sans acide urique , à savoir le mélange de 7 composés antioxydants suivants : hydrosulfure de sodium (Na₂S), L-Cystéine, acide ascorbique, glutathion, et trois antioxydants supplémentaires, à savoir la catalase, l'ubiquinol et l'acide lipoïque. Ces milieux ont été testés pour la culture des 13 bactéries anaérobies fastidieuses incluant les bactéries anaérobies citées ci-dessus et 20 bactéries aérobies les plus communément rencontrées en routine hospitalière décrites ci-après.

La présente invention fournit un milieu de culture liquide polyvalent pour culture en atmosphère aérobie de bactéries anaérobies ou bactéries aérobies comprenant un milieu de culture de base pour bactéries aérobies et anaérobies caractérisé en ce qu'il comporte en outre le mélange de composés antioxydants suivants: hydrosulfure de sodium (Na₂S), L-Cystéine, acide ascorbique, glutathion, catalase, ubiquinol et acide lipoïque.

Plus particulièrement, le milieu de culture selon l'invention comprend les dits composés antioxydants aux quantités et proportions pondérales suivantes pour 1L :

| | |
|---|---|
| - Hydrosulfure de sodium : | au moins 0,25 g (0,025%), de préférence de 0,25 g à 0,5 g (0,025 à 0,05 %) |
| - L-Cystéine : | au moins 0,25 g (0,025%), de préférence de 0,25g à 0,5 g (0,025 à 0,05 %) |
| - Acide ascorbique : | au moins 0,5 g (0,05%), de préférence de 0,5 à 1 g (0,05 à 0,1 %) |
| - Glutathion : | au moins 0,1 g (0,01%), de préférence de 0,1 à 0,5 g (0,01 à 0,05 %) |
| - Catalase : | au moins 0,06 g (0,006%), de préférence de 0,06 à 0,16 g (0,006 à 0,016 %) |
| - Ubiquinol : | au moins 0,06 g (0,006%), de préférence de 0,06 à 0,16 g (0,006 à 0,016 %) |
| - Acide lipoïque : | au moins 0,01 g (0,001%), de préférence de 0,01 à 0,015 g (0,001 à 0,0015 %) |

Plus particulièrement encore, le milieu de culture selon l'invention comprend les composants nutritifs suivants dans le dit milieu de culture de base :
- plusieurs sources de carbone et d'azote, de préférence choisies parmi un extrait de levure, un sel d'acétate et une peptone trypsique,
- une source de phosphore, de préférence un sel de phosphate,
- au moins un sucre, et
- au moins un sel de métaux choisis parmi K, Mg, Na et Ca, de préférence NaCl,
- ainsi que
- au moins une substance tampon régulateur de pH pour ajuster le pH de 7 à 8, de préférence K2HPO4 ou NaHCO3 pour ajuster le pH à 7,5, et
- au moins une vitamine ou facteur de croissance.

Plus particulièrement encore, ledit milieu de culture de base est un milieu acellulaire conventionnel de bactérie comprenant des composants nutritifs choisis parmi un extrait de broyat ou lysat de tissu pluricellulaire, un digestat enzymatique, notamment un digestat enzymatique de caséine, soja et/ou de tissu animal, une peptone, un extrait de levure, un sucre tel que dextrose ou glucose, un sel NaCl et/ou Na₂PO₄.

Plus particulièrement encore, ledit milieu de culture de base est un milieu acellulaire choisi parmi un milieu axénique constitué de substances chimiques ou biologiques défini qualitativement et quantitativement, et un milieu acellulaire comprenant un extrait de broyat ou lysat de tissu pluricellulaire.

Plus particulièrement encore, ledit milieu de culture est un milieu acellulaire conventionnel de bactérie anaérobie, notamment un milieu comprenant des composants choisis parmi un extrait de broyat ou lysat de tissu pluricellulaire, un digestat enzymatique, notamment un digestat enzymatique de caséine, soja et/ou de tissu animal, une peptone, un extrait de levure, un sucre tel que dextrose ou glucose, un sel NaCl et/ou Na₂PO₄.

Plus particulièrement encore, ledit milieu de culture de base est un milieu conventionnel de culture de bactéries anaérobies tel que les milieux dits bouillon de type cœur-cervelle, milieux Columbia à 5% de sang de mouton ou milieu Schaedler tels que décrits ci-après. D'autres milieux conventionnels appropriés sont les milieux Brucella ou Wilkins-Chagren. De tels milieux de culture acellulaires sont bien connus de l'homme de l'art.

On peut utiliser en particulier des milieux de culture polyvalents pour microorganismes anaérobies, notamment le milieu de Schaedler, ledit milieu étant complémenté en composés hydrocarbonés, de préférence de l'amidon et de(s) sucre(s), et en dit(s) composé(s) antioxydant(s).

Plus particulièrement encore, ledit milieu de culture de base est un milieu de culture liquide pour culture de bactéries anaérobies dans un échantillon sanguin, selle, crachat ou sécrétion vaginale.

Plus particulièrement encore, ledit milieu de culture de base comprend les composants nutritifs suivants:
- Hydrolisat de caséine ;
- Protéose peptone ;
- Extrait de levure ;
- Chlorure de sodium (NaCl) ;
- Glucose, et
- Un facteur de croissance : α-cétoglutarate.

Plus particulièrement encore, ledit milieu de culture de base comprend les composants suivants dans les quantités et proportions pondérales suivantes pour 1L :

| | |
|---|---|
| - Hydrolisat de caséine: | 15 g (1,5 %) |
| - Proteose peptone: | 15 g (1,5 %) |
| - Extrait de levure: | 10 g (1 %) |
| - α-cétoglutarate : | 2 g (0,2 %) |
| - Chlorure de sodium (NaCl) : | 5 g (0,5 %) |
| - Glucose: | 10 g (1 %) |
| - Dipotassium phosphate (K₂HPO₄) : | 0,83 g (0,083 %) |
| - Hydrosulfure de sodium (Na₂S) : | 0,5 g (0,05 %) |
| - L-Cystéine : | 0,5 g (0,05 %) |
| - Acide ascorbique : | 1 g (0,1 %) |
| - Glutathion : | 0,1 g (0,01 %) |
| - Catalase : | 0,16 g (0,016 %) |
| - Ubiquinol : | 0,16 g (0,016 %) |
| - Acide lipoïque : | 0,010 g (0,001 %) |

Le pH de la solution étant ajusté à 7,5±0,2 avec du KOH 10M.

La présente invention a également pour objet un procédé de culture *in vitro* d'une bactérie aérobie ou anaérobie sous atmosphère aérobie avec un milieu de culture selon l'invention.

Plus particulièrement, on réalise une culture d'une dite bactérie dans un échantillon de sang, selle, crachat ou sécrétion vaginale.

Plus particulièrement encore, on réalise une culture d'une dite bactérie anaérobie stricte fastidieuse choisie parmi *Akkermansia muciniphila, Bacteroides fragilis, Bacteroides nordii, Bacteroides thetaiotaomicron, C*/*ostridium beijeirinckii, C*/*ostridium butyricum, Clostridium massilioamazoniensis, C*/*ostridium irregulare, Finegoldia magna, Propionibacterium acnes et Propionibacterium avidum, Prevotella denticola et Prevotella histicola.*

Plus généralement, le milieu selon la présente invention permet aussi la culture de bactéries dont la croissance est sensible à la tension en oxygène et notamment des bactéries qui tolèrent mal des tensions élevées d'oxygène et pour lesquelles une croissance optimale de la dite bactérie requière une atmosphère d'incubation à teneur en oxygène relativement réduite par rapport à la teneur en oxygène de l'air en l'absence de milieu de culture spécifique tel qu'un milieu selon la présente invention. On distingue donc parmi les bactéries sensibles à l'oxygène :
- les bactéries microaérophiles c'est-à-dire qu'elles ne sont pas capables de cultiver sous une atmosphère comprenant la concentration d'oxygène ambiante qui est de environ 21%, notamment entre 1% et 20%, le plus communément à environ 2-2,5%, et
- les bactéries anaérobies strictes c'est-à-dire qu'elles ne sont pas capables de cultiver en présence d'oxygène ou dans des concentrations inférieures aux concentrations de microaérophilie, notamment strictement inférieure à 1%, le plus communément inférieure à 0.1%, idéalement 0%. Pour cultiver les bactéries anaérobies strictes, en l'absence de milieu de culture spécifique tel qu'un milieu selon la présente invention, il faut soit les cultiver dans des étuves ne comportant pas d'oxygène, soit dans des tubes qui ont été désoxygénés et elles ne poussent alors qu'au fond du tube.

Parmi les bactéries anaérobies strictes, on cite plus particulièrement les bactéries extracellulaires, c'est-à-dire des bactéries qui ne peuvent vivre qu'à l'extérieur de cellules.

Parmi les bactéries cultivables en atmosphère microaérophile, on distingue plus particulièrement, les bactéries intracellulaires, mais aussi des bactéries extracellulaires. On entend ici par «bactérie intracellulaire», une bactérie qui a la capacité de se multiplier au sein d'une cellule hôte. Les bactéries intracellulaires, ayant la faculté de croître dans certaines conditions dans des milieux acellulaires, sont dénommées "bactéries intracellulaires facultatives".

On entend ici par :
- «bactérie intracellulaire facultative », une bactérie qui a la capacité de se multiplier au sein d'une cellule hôte en milieu acellulaire, et
- «bactérie extracellulaire », une bactérie qui n'a pas la capacité de se multiplier au sein d'une cellule hôte et se cultive exclusivement en milieu acellulaire.

On entend ici par «milieu de culture acellulaire», un milieu de culture qui ne comprend pas de cellules entières notamment qui ne comprend pas de cellules hôtes entières au sein desquelles la dite bactérie peut se multiplier, lorsque ladite bactérie est intracellulaire ou intracellulaire facultative. On comprend que les cellules entières doivent être vivantes pour permettre une multiplication de la bactérie en leur sein.

Plus particulièrement, la présente invention concerne la culture de bactéries anaérobies et la culture de bactéries microaérophiles intracellulaires.

Plus particulièrement encore, on peut cultiver la dite bactérie dans une dite atmosphère d'incubation comprenant une proportion molaire en oxygène supérieure à la tension maximale tolérée en l'absence de composé antioxydant pour un même niveau de croissance dans une même durée de culture.

En pratique, plus particulièrement encore, on peut cultiver la dite bactérie dans une dite atmosphère d'incubation comprenant une proportion molaire en oxygène supérieure à 2.5% et inférieure ou égale à 20%
Avantageusement toutefois, on cultive lesdites bactéries selon la présente invention dans une atmosphère comprenant une teneur en oxygène supérieure à 5%, notamment dans de l'air contenant 5% de CO2 (soit une teneur en oxygène inférieure à 16%).

De préférence, on cultive lesdites bactéries selon la présente invention dans une atmosphère d'air.

Les bactéries anaérobies peuvent être des bactéries anaérobies strictes ou bactéries anaérobies facultatives aussi dénommées aéro-anaérobies c'est-à-dire des bactéries anaérobies qui tolèrent de l'oxygène mais n'en n'ont pas besoin pour croitre ou bactéries aérobies qui supportent l'absence d'oxygène pour croître.

Parmi les bactéries anaérobies strictes, on cite plus particulièrement les autres bactéries anaérobies strictes appartenant aux genres *Acidaminococcus, Alistipes, Anaerococcus, Anaerosalibacter, Amazonia, Atopobium, Bifidobacterium, Blautia, Bacteroides, Barnesiella, Clostridium, Collinsella, Dielma, Eggerthella, Finegoldia, Flavonifractor, Fusobacterium, Gordonibacter, Guyana, Holdemania, Odoribacter, Parabacteroides, Parvimonas, Prevotella, Peptostreptococcus, Peptoniphilus, Porphyromonas, Prevotella, Solobacterium, Tissierella, Turicibacter, Ruminococcus et Veillonella.*

Parmi les bactéries anaérobies facultatives, on cite plus particulièrement les autres bactéries anaérobies facultatives appartenant aux genres *Actinomyces, Aerococcus, Aeromonas, Aneurinibacillus, Bacillus, Bartonella, Cedecea, Citrobacter, Corynebacterium, Derambacter, Eikenella, Enterobacter, Enterococcus, Escherichia, Eubacterium, Gardnerella, Gemella, Granulicatella, Hafnia, Haemophilus Kingella, Klebsellia, Lactobacillus, Lactococcus, Lysinibacillus, Morganella, Paenibacillus, Pasteurella, Pediococcus, Propionibacterium, Proteus, Providencia, Serratia, Raoultella, Rothia, Staphylococcus, Streptococcus et Weissella.*

D'autres caractéristiques et avantages de la présente invention ressortiront mieux à la lecture de la description détaillée qui va suivre, faite de manière illustrative et non limitative.

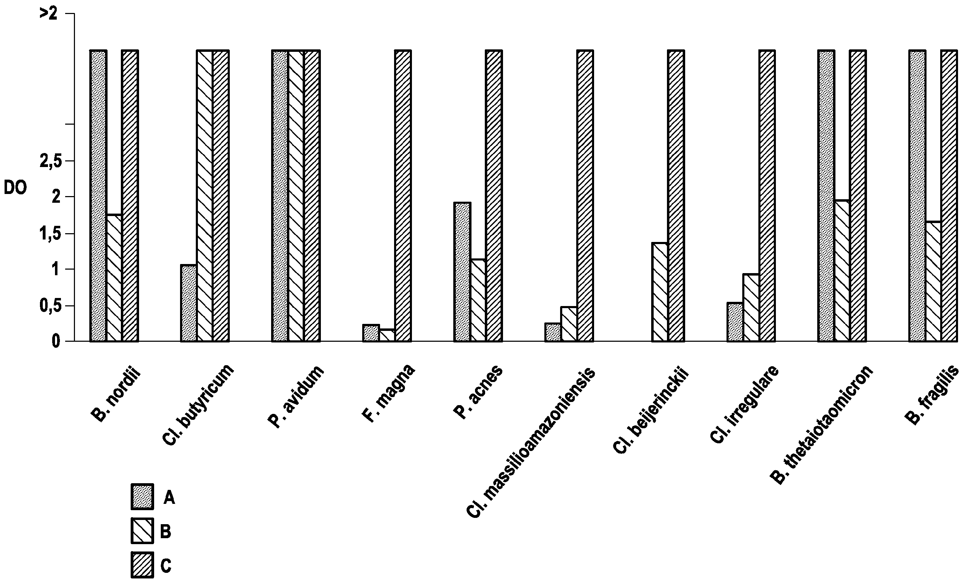

La figure 1 représente la comparaison de la croissance des bactéries anaérobies dans les trois milieux A, B et C testés à l'exemple 3.

Afin de comparer l'efficacité des différents milieux testés ci-après de culture, on a réalisé la culture de 10 bactéries anaérobies et 20 bactéries aérobies les plus communément rencontrées en routine hospitalière en utilisant les méthodes de références (utilisation des flacons anaérobies pour cultiver les bactéries anaérobies et les flacons aérobies pour cultiver les bactéries aérobies). La liste de ces bactéries ainsi que leurs conditions de cultures sont détaillées dans le tableau 1 ci-après. Ces souches sont dans la collection de souches du laboratoire, la CSUR (Collection de Souches de l'Unité Rickettsies).

**Tableau 1. Liste des microorganismes utilisés dans cette étude**

| **Bactéries aérobies** | **Bactéries anaérobies** |
|---|---|
| *Staphylococcus aureus* P412 | *Bacteroides nordii* P3192 |
| *Enterobacter aerogenes* P455 | *Propionibacterium avidum* P3346 |
| *Escherichia coli* P430 | *Clostridum irregulare* P1913 |
| *Klebsiella oxytoca* P1603 | *Clostridum massilioamazoniensis* P1360 |
| *Streptococcus agalactiae* P4162 | *Clostridum butyricum* P344 |
| *Serratia marcescens* P587 | *Clostridum beijerinckii* P883 |
| *Enterococcus faecalis* P2282 | *Bacteroides thetaiotaomicron* P575 |
| *Proteus mirabilis* P2711 | *Propionibacterium acnes* P637 |
| *Pseudomonas aeruginosa* P2378 | *Finegoldia magna* P588 |
| *Streptococcus mitis* P4157 | *Bacteroides fragilis* P444 |
| *Staphylococcus epidermidis* P4227 | *Akkermansia muciniphila* P4531 et P3284 |
| *Morganella morganii* P4358 | *Prevotella histicola* P1055 |
| *Citrobacter freundii* P4462 | *Prevotella denticola* P1023 |
| *Enterobacter cloacae* P4822 | |
| *Bacillus circulans* P655 | |
| *Neisseria meninigitidis* P782 | |
| *Streptococcus pneumoniae* P3582 | |
| *Staphylococcus hominis* P3863 | |
| *Acinetobacter baumanii* P1976 | |
| *Haemophilus influenzae* P4027 | |

Pour tester la croissance des bactéries dans les différents milieux, en atmosphère aérobie, les inventeurs ont vidé des flacons d'hémoculture et les ont remplis avec le milieu de culture. Une fois le mélange homogène et les poudres dissoutes, le milieu a été filtré à travers un filtre de 0,2 µm d'épaisseur, afin de le stériliser. 40 millilitres de ce milieu a ensuite été transféré dans chacun des flacons de culture, contenant une atmosphère aérobie.

Afin d'ensemencer les flacons de culture avec les bactéries, les inventeurs ont dilué pour chaque bactérie une colonie, ce qui correspond à environ 10⁶ bactéries, dans 1 millilitre du même milieu de culture. Après avoir vortexé le tube contenant ce mélange, afin de diluer correctement la colonie dans le liquide, les inventeurs ont injecté le millilitre dans le flacon de culture à l'aide d'une seringue et d'une aiguille stérile et ont mis le flacon à incuber à 37°C pendant 72 heures pour les bactéries anaérobies et 24 heures pour les bactéries aérobies. Le même protocole a été réalisé pour chaque bactérie. Un millilitre de milieu contenant 10⁶ bactéries ayant été introduit dans un flacon de culture de 40 millilitres, la quantité finale de bactéries dans le flacon de culture est de 2,5x10⁴ bactéries.

Les bactéries aérobies utilisées pour réaliser cette étude sont cultivées et repiquées sur le milieu gélosé Columbia agar + 5% de sang de mouton incubées dans une étuve maintenue à température constante de 37°C sans ajout de CO₂. Les bactéries anaérobies ont été cultivées sur ce même milieu et déposées dans des jarres anaérobies équipées de générateurs d'anaérobiose. Après leur croissance, les bactéries aérobies et anaérobies ont été identifiées par spectrométrie de masse de type MALDI-TOF comme précédemment décrit [4].

Les inventeurs ont étudié la croissance des souches de bactéries aérobies, et des souches de bactéries anaérobies, du tableau 1, dans 3 milieux liquides différents.

Pour l'inoculation des bactéries dans les flacons d'hémocultures, les inventeurs ont réalisés des suspensions bactériennes de chacune des souches testées. Pour cela, 1 mL de chaque milieu de culture respectivement a été distribué dans chaque tube eppendorf, sachant qu'il y a trois tubes eppendorf par bactérie (un tube pour chaque milieu différent).

Pour chaque bactérie, une colonie, correspondant à environ 10⁶ bactéries, a été introduite dans le tube eppendorf contenant 1 mL du milieu de culture. Les inventeurs ont vortexé le tube afin d'obtenir un mélange homogène de la colonie dans le milieu de culture. Ils ont ensuite inoculé la bactérie dans le flacon de culture à l'aide d'une seringue et d'une aiguille stérile.

Le flacon de culture a ensuite été mis à incuber pendant 72 heures à 37°C.

Ce protocole a été réalisé pour chacune des bactéries et chacun des milieux.

Sur la figure 1, on rapporte les résultats de la croissance des bactéries anaérobies en aérobie. Après 72 heures d'incubation, les inventeurs ont mesuré la densité optique (DO) de chaque flacon de culture. Pour avoir une mesure objective, ils ont fait le témoin sur du milieu de culture non inoculé, afin de mettre en évidence la croissance bactérienne.

La DO a été mesurée pour chacune des bactéries dans chacun des milieux. Par exemple, pour le milieu testé, le témoin ou zéro a été fait avec ce même milieu non inoculé, puis la mesure de DO a été faite pour chacune des bactéries. Le témoin est toujours fait avec le milieu qui a été introduit dans la culture dont on veut mesurer la DO. L'appareil de mesure (Fisher scientific, Illkirch, France) ne permet pas de mesurer des DO au-delà de 2 qui correspondent à des fortes croissances de bactéries.

### Exemple 1

Les inventeurs ont testé différents antioxydants, ainsi que différents mélanges et sélectionné le mélange décrit ci-dessus, dans la formule du milieu de culture selon l'invention.

Afin de sélectionner le meilleur mélange d'antioxydants, les inventeurs ont testé un total de 13 antioxydants.

Dans un premier temps, les inventeurs ont testé le mélange de base d'antioxydants, déjà connu, à savoir l'hydrosulfure de sodium (Na₂S), la L-Cystéine, l'acide ascorbique et le glutathion, avec la peroxydase. Puis, ce même mélange avec la super oxyde dismutase et enfin avec l'oxydase. Cependant, les inventeurs n'ont pas observé d'amélioration dans la croissance des bactéries anaérobies en aérobie.

Ils ont alors testé 6 autres antioxydants avec le mélange de base décrit ci-dessus. Pour cela, ils ont expérimenté 58 combinaisons différentes de tous ces antioxydants (cf tableaux 2A-2C ci-après).

Ils ont testé ces mélanges en milieu liquide, sur des plaques de culture 24 puits (Greiner Bio-One International, Kremsmünster, Autriche) avec plusieurs bactéries aérobies et anaérobies dont la bactérie *Finegoldia magna,* car c'est la bactérie qui avaient montré le plus de difficulté à croitre lors des tests réalisés dans le laboratoire sur le milieu Versatrek™ avec antioxydants.

Pour chaque plaque, les inventeurs ont utilisé trois puits comme témoin négatif, ces puits contenaient 1 mL du milieu R-medium avec les antioxydants de base mais sans bactéries et trois puits contenant 1 mL du même milieu mais avec ensemencement de la bactérie *Finegoldia magna* pour montrer qu'il n'y avait pas une croissance optimale de cette bactérie dans le mélange initial. De plus, pour avoir un témoin positif, les inventeurs ont dupliqué chaque plaque et ont mis le duplicata en condition d'anaérobie, les bactéries testées étant anaérobie stricte.

Afin de tester chaque antioxydant et différentes combinaisons de ces antioxydants, les inventeurs ont préparé des solutions mères des 6 antioxydants testés en plus du mélange d'antioxydants de base.

Du milieu R-medium avec les antioxydants de base a été distribué dans chaque puits et à ces puits, un certain volume de la solution mère de chaque antioxydant, correspondant à la concentration souhaitée, a été ajouté dans les puits. La répartition des antioxydants dans les puits a suivi le tableau 2. Le volume de R-medium avec les antioxydants de base, ajouté dans les puits est la différence entre les 1 mL, souhaité comme volume final et les volumes des antioxydants ajoutés. En effet, pour ne pas créer de biais, les inventeurs ont souhaité que le volume final, après ajout des antioxydants, dans chaque puits soit de 1 mL.

Les solutions mères des 6 antioxydants ont été préparées dans les quantités et proportions pondérales suivantes:
- Catalase : Dissolution de 100 mg de catalase dans 100 mL d'eau distillée. Obtention d'une solution mère à une concentration de 1 g/L. Pour obtenir une concentration finale, dans chaque puits, de 160 mg/L, 160 µL de la solution mère ont été ajoutée dans les puis concernés.

- Ubiquinol : Dissolution de 100 mg d'ubiquinol dans 100 mL d'eau distillée. Obtention d'une solution mère à une concentration de 1 g/L. Pour obtenir une concentration finale, dans chaque puits, de 160 mg/L, 160 µL de la solution mère ont été ajoutée dans les puis concernés.
- BHT : Dissolution de 50 mg de BHT dans 10 mL d'éthanol car le BHT n'est pas soluble dans l'eau. Obtention d'une solution mère à une concentration de 5 g/L. Pour obtenir une concentration finale, dans chaque puits, de 100 mg/L, 20 µL de la solution mère ont été ajoutée dans les puis concernés.
- Vitamine E : Dissolution de 10 mg de vitamine E dans 10 mL d'éthanol car la vitamine E n'est pas soluble dans l'eau. Obtention d'une solution mère à une concentration de 1 g/L. Pour obtenir une concentration finale, dans chaque puits, de 100 µM soit 43 mg/L, 43 µL de la solution mère ont été ajoutée dans les puis concernés.
- Acide lipoïque : Dissolution de 100 mg d'acide lipoïque dans 10 mL d'éthanol car l'acide lipoïque n'est pas soluble dans l'eau. Obtention d'une solution mère à une concentration de 10 g/L. Pour obtenir une concentration finale, dans chaque puits, de 100 mg/L, 10 µL de la solution mère ont été ajoutée dans les puis concernés.
- NAD : Dissolution de 10 mg de NAD dans 10 mL d'eau distillée. Obtention d'une solution mère à une concentration de 1 g/L. Pour obtenir une concentration finale, dans chaque puits, de 15 mg/L, 15 µL de la solution mère ont été ajoutée dans les puits concernés.

Les inventeurs ont mesuré le pH de chaque solution mère et l'ont ajusté au pH de 7,3±0,2 quand cela était nécessaire.

Les inventeurs ont préparé une suspension de la bactérie *Finegoldia magna* concentrée à 10⁴ et ont prélevé 100 µL de cette solution qu'ils ont introduit dans chacun des puits, afin d'obtenir une concentration bactérienne finale de 10³ bactéries par millilitre. Ils ont ensuite incubé les boites témoin positif en anaérobie et les plaques testées en aérobie, durant 72h et à 37°C.

Après 72 h d'incubation, les inventeurs ont observé les plaques et sélectionné la combinaison en antioxydants qui avait entrainé la turbidité la plus importante du milieu liquide. Ils n'ont pas mesuré de manière quantitative la croissance bactérienne, ils se sont basés sur une observation d'un trouble éventuel reflétant la croissance bactérienne. Certains autres puits ont montré un léger trouble mais rien de comparable à la turbidité surprenante de la combinaison la plus favorable sélectionnée des 7 antioxydants du tableau 2B avec ajout de - catalase, ubiquinol et acide lipoïque au mélange de base d'antioxydants, déjà connu, à savoir l'hydrosulfure de sodium (Na₂S), la L-Cystéine, l'acide ascorbique et le glutathion.

La combinaison la plus favorable à la croissance de cette bactérie a été le mélange antioxydants de base comprenant les antioxydants, l'hydrosulfure de sodium (Na₂S), la L-Cystéine, l'acide ascorbique et le glutathion, combiné avec les 3 antioxydants supplémentaires : l'ubiquinol, la catalase et l'acide lipoïque.

Tableaux 2A-2C : Combinaisons des différents antioxydants, avec pour milieu de base le R-medium avec les 4 antioxydants de base (hydrosulfure de sodium (Na₂S), L-Cystéine, acide ascorbique, glutathion) (3 tableaux 2A, 2B et 2C, chaque tableau représentant une plaque).

Dans les tableaux 2A à 2C :
- BHT = Butylhydroxytoluène
- NAD = Nicotinamide adénine dinucléotide

Le BHT est un antioxydant synthétique, E321 listé comme antioxydant au Codex alimentarius. [5]).

Le NAD est un antioxydant endogène Co-Enzyme 1 A de Niacine présent dans toute cellule vivante de mammifère.

**Tableau 2A = ajout de 2 antioxydants**

| Témoin négatif | Témoin négatif | Témoin négatif | Base R-medium sans acide urique avec F.magna | Base R-medium sans acide urique avec F.magna | Base R-medium sans acide urique avec F.magna |
|---|---|---|---|---|---|
| Catalase 160 mg/L | Ubiquinol 160 mg/L | BHT 100 mg/L | Vitamine E 100 µM soit 43 mg/L | NAD 15 mg/L | Acide lipoïque 10 mg/L |
| Catalase Ubiquinol | Catalase BHT | Catalase Vitamine E | Catalase NAD | Catalase Acide lipoïque | Ubiquinol BHT |
| Ubiquinol Vitamine E | Ubiquinol NAD | Ubiquinol Acide lipoïque | BHT Vitamine E | BHT NAD | BHT Acide lipoïque |

**Tableau 2B = ajout de 3 ou 4 antioxydants**

| Témoin négatif | Témoin négatif | Témoin négatif | Base R-medium sans acide urique avec F.magna | Base R-medium sans acide urique avec F.magna | Base R-medium sans acide urique avec F.magna |
|---|---|---|---|---|---|
| - Vitamine E - NAD - BHT | - Vitamine E - Acide lipoïque ^{..} - NAD | - Catalase - Ubiquinol - BHT | - Catalase - Ubiquinol - Vitamine E | - Catalase - Ubiquinol - NAD | **- Catalase - Ubiquinol - Acide lipoïque** |
| - Ubiquinol - BHT - Vitamine E | - Ubiquinol - BHT - NAD | - Ubiquinol - BHT - Acide lipoïque | - Ubiquinol - Vitamine E - NAD | - Ubiquinol - Vitamine E - Acide lipoïque | - Ubiquinol - NAD - Acide lipoïque |
| - Catalase - Ubiquinol - Acide lipoïque - BHT | - Catalase - BHT - Vitamine E - NAD | - Catalase - BHT - Vitamine E - Acide lipoïque | - Catalase - Vitamine E - NAD - Acide lipoïque | - Ubiquinol - BHT - Vitamine E - NAD | - Ubiquinol - BHT - Vitamine E - Acide lipoïque |

**Tableau 2C = ajout de 5 antioxydants**

| Témoin négatif | Témoin négatif | Témoin négatif | Base R-medium sans acide urique avec F.magna | Base R-medium sans acide urique avec F.magna | Base R-medium sans acide urique avec F.magna |
|---|---|---|---|---|---|
| - Ubiquinol - BHT - NAD - Acide lipoïque | - Ubiquinol - Vitamine E - NAD - Acide lipoïque | - BHT - Vitamine E - NAD - Acide lipoïque | - Catalase - Ubiquinol - BHT - Vitamine E | - Catalase - Ubiquinol - BHT - Vitamine E - Acide lipoïque | - Catalase - Ubiquinol - BHT - NAD - Acide lipoïque |
| - Catalase - Ubiquinol - NAD - Acide lipoïque Vitamine E | - Catalase - Vitamine E - BHT - NAD - Acide lipoïque | - Ubiquinol - Vitamine E - BHT - NAD - Acide lipoïque | - NAD - Acide lipoïque - Catalase - Ubiquinol - Vitamine E - BHT | | |

### Exemple 2

Afin de démontrer que cette meilleure croissance des bactéries anaérobies testées, en aérobie, était due seulement au mélange d'antioxydants et non pas au milieu de culture de base utilisé, les inventeurs ont testé l'ancienne version d'antioxydants (hydrosulfure de sodium (Na₂S), L-Cystéine, acide ascorbique, glutathion) dans le milieu Versatrek™ commercialisé (société I2a, Montpelier, France) et la nouvelle version d'antioxydants dans ce même milieu (hydrosulfure de sodium (Na₂S), L-Cystéine, acide ascorbique, glutathion, catalase, ubiquinol et acide lipoïque.

Concernant le milieu Versatrek™ + 4 antioxydants de l'art antérieur, les flacons VersaTREK™ supplémenté (société I2a, Montpelier, France) ont été supplémentés en composés antioxydants conventionnels suivants dans les quantités et proportions pondérales suivantes pour 25 mL :

| | |
|---|---|
| - Hydrosulfure de sodium (Na₂S) : | 0,25 g (0,025 %) |
| - Acide urique : | 0,2 g (0,02 %) |
| - Acide ascorbique : | 0,5 g (0,05 %) |
| - Glutathion : | 0,05 g (0,005 %) |

Concernant le milieu Versatrek + 7 antioxydants selon l'invention, les flacons VersaTREK™ supplémenté (société I2a, Montpelier, France) ont été supplémentés en composés antioxydants conventionnels suivants dans les quantités et proportions pondérales suivantes pour 1L :

| | |
|---|---|
| - Hydrosulfure de sodium (Na₂S) : | 0,5 g (0,05 %) |
| - Acide urique : | 0,4 g (0,04 %) |
| - Acide ascorbique : | 1 g (0,1 %) |
| - Glutathion : | 0,1 g (0,01 %) |
| - Catalase : | 0,16 g (0,016 %) |
| - Ubiquinol : | 0,16 g (0,016 %) |
| - Acide lipoïque : | 0,010 g (0,001 %) |

Les bactéries anaérobies citées précédemment ont été testées dans ces deux milieux et incubées à 37°C pendant 72h et les inventeurs ont pu observer une meilleure croissance de ces bactéries dans le mélange milieu Versatrek + 7 antioxydants.

Les inventeurs ont pu en déduire que le milieu nutritionnel de base utilisé importe peu, si le mélange des 7 antioxydants est ajouté au milieu, les bactéries anaérobies auront une meilleure croissance en aérobie.

### Exemple 3 : Tests comparatifs sur la croissance de bactéries anaérobies fastidieuses courantes.

On évalue ci-après les performances de croissance des différents milieux de culture en culture incubés dans une étuve à 37°C, à savoir le milieu du flacon universel de culture selon la présente invention et des milieux connus comparatifs comprenant un milieux de culture en aérobie et anaérobie classiques BD BACTEC™ à base de bouillon Trypticase soja enrichi (milieu A sur la figure 1) et un milieu de flacons d'hémoculture VersaTREK™ supplémenté avec des antioxydants décrits ci-après (milieu B sur la figure 1) (référence 191401, VersaTREK REDOX 1 aérobie de la société I2a, Montpelier, France) [3].

Le milieu de culture selon l'invention dénommé « R medium » (milieu C sur la figure 1) comprend les composants suivants dans les quantités et proportions pondérales suivantes pour 1L :

**Milieu de base:**

| | |
|---|---|
| - Hydrolisat de caséine: | 15 g (1,5 %) |
| - Proteose peptone: | 15 g (1,5 %) |
| - Extrait de levure: | 10 g (1 %) |
| - α-cétoglutarate : | 2 g (0,2 %) |
| - Chlorure de sodium (NaCl) : | 5 g (0,5 %) |
| - Glucose: | 10 g (1 %) |
| - Dipotassium phosphate (K₂HPO₄) : | 0,83 g (0,083%) |

**Composés antioxydants :**

| | |
|---|---|
| - Hydrosulfure de sodium (Na₂S) : | 0,5 g (0,05 %) |
| - L-Cystéine : | 0,5 g (0,05 %) |
| - Acide ascorbique : | 1 g (0,1 %) |
| - Glutathion : | 0,1 g (0,01 %) |
| - Catalase : | 0,16 g (0,016 %) |
| - Ubiquinol : | 0,16 g (0,016 %) |
| - Acide lipoïque : | 0,010 g (0,001 %) |

Les deux autres milieux testés, à savoir les flacons de culture anaérobies BD BACTEC™ et les flacons VersaTREK™ supplémenté (société I2a, Montpelier, France) supplémentés en composés antioxydants conventionnels suivants dans les quantités et proportions pondérales suivantes pour 25 mL :

| | |
|---|---|
| - Hydrosulfure de sodium (Na₂S) : | 0,25 g (0,025 %) |
| - Acide urique : | 0,2 g (0,02 %) |
| - Acide ascorbique : | 0,5 g (0,05 %) |
| - Glutathion : | 0,05 g (0,005 %) |

Les résultats ont montré que pour un même temps d'incubation, la DO mesurée pour les bactéries anaérobies incubées en atmosphère aérobie est supérieure avec le milieu de culture de la présente invention (milieu C de la figure 1), par rapport aux milieux VersaTREK™ supplémenté en antioxydants (milieu B de la figure 1), et le flacon de culture avec le milieu de culture anaérobie BD BACTEC™ (milieu A de la figure 1), utilisé communément comme montré sur la figure 1.

La figure 1 représente les DO illustrant la croissance des bactéries anaérobies dans les trois milieux A, B et C testés à 72h d'incubation à 37°C pour les 10 bactéries anaérobies fastidieuses suivantes communément rencontrées en pratiques hospitalières : *Bacteroides nordii, Propionibacterium avidum, Clostridum irregular, Clostridum massilioamazoniensis, Clostridum butyricum, Clostridum beijerinckii, Bacteroides thetaiotaomicron, Propionibacterium acnes, Finegoldia magna et Bacteroides fragilis.*

Des résultats similaires ont été obtenus pour les bactéries aérobies dans les trois milieux testés.

### Exemple 4 : Test de croissance de différentes autres bactéries fastidieuses retrouvées chez l'homme dans la cavité orale ou le tractus gastro-intestinal

Le milieu de culture de la présente invention R medium et le milieu Versatek™ supplémenté restent identiques à ceux décrits dans l'exemple 3.

Les inventeurs on testés 2 souches de *Prevotella : P. denticola et P. histicola et 2* souches *d'Akkermansia muciniphila* P3284 et P4531.

Ces bactéries sont anaérobies strictes et sont retrouvées chez l'homme dans la cavité orale ou le tractus gastro-intestinal.

De la même façon que décrit précédemment, les inventeurs ont réalisés des suspensions bactériennes de chacune des souches testées. Pour cela, 1 mL de milieu de culture a été distribué dans chaque tube eppendorf, sachant qu'il y a un tube eppendorf par bactérie.

Pour chaque bactérie, une colonie, correspondant à environ 10⁶ bactéries, a été introduite dans le tube eppendorf contenant 1 mL du milieu de culture. Les inventeurs ont vortexé le tube afin d'obtenir un mélange homogène de la colonie dans le milieu de culture. Ils ont ensuite inoculé la bactérie dans la bouteille d'hémoculture à l'aide d'une seringue et d'une aiguille stérile.

Le flacon de culture a ensuite été mis à incuber pendant 5 jours à 37°C.

Les 4 souches des 3 bactéries ont eu une croissance dans le milieu selon l'invention R-medium et pas de croissance dans le milieu Versatek™ supplémenté décrit à l'exemple 3.

### BIBLIOGRAPHIE

(1) Strobel H. Basic laboratory culture methods for anaerobic bacteria. In : Jonathan R. Mielenz, editor. 2009 p 247-248
(2) N. Dione, S. Khelaifia, B. La Scola, J.C. Lagier, D. Raoult. A quasi-universal medium to break the aerobic/anaerobic bacterial culture dichotomy in clinical microbiology. G. Greub, editor. Clin Microbiol Infect. 2016 Jan;22(1):53-8. January 2016.
(3) Grégory Dubourg, Elodie Guilhot, Saber Khelaifia, Enora Tomei, Jean-Paul Casalta, Pierre-Yves Lévy, Hervé Tissot-Dupont, Didier Raoult.. Evaluation of the Versatrek universal vial in the diagnosis of bacteremia: a preliminary report. (submitted).
(4) Seng P, Drancourt M, Gouriet F, La Scola B, Fournier PE, Rolain JM, Raoult D. Ongoing revolution in bacteriology: routine identification of bacteria by matrix-assisted laser desorption ionization time-of-flight mass spectrometry. Clin Infect Dis. 2009 Aug 15;49(4):543-51.
(5) Codex alimentarius, normes alimentaires internationale. CAC/GL 36-1989. Page 10

## Revendications

1. Milieu de culture liquide polyvalent pour culture en atmosphère aérobie de bactéries anaérobies ou bactéries aérobies comprenant un milieu de culture de base pour bactéries aérobies et anaérobies **caractérisé en ce qu'**il comporte en outre le mélange de composés antioxydants suivants: hydrosulfure de sodium (Na₂S), L-Cystéine, acide ascorbique , glutathion, catalase, ubiquinol et acide lipoïque.

2. Milieu de culture selon la revendication 1, **caractérisé en ce qu'**il comprend les dits composés antioxydants aux quantités et proportions pondérales suivantes pour 1L :
| | |
|---|---|
| - Hydrosulfure de sodium | : au moins 0,25 g (0,025%), de préférence de 0,25 g à 0,5 g (0,025 à 0,05 %) |
| - L-Cystéine : | au moins 0,25 g (0,025%), de préférence de 0,25 g à 0,5 g (0,025 à 0,05 %) |
| - Acide ascorbique : | au moins 0,5 g (0,05%), de préférence de 0,5 à 1 g (0,05 à 0,1 %) |
| - Glutathion : | au moins 0,1 g (0,01%), de préférence de 0,1 à 0,5 g (0,01 à 0,05 %) |
| - Catalase : | au moins 0,06 g (0,006%), de préférence de 0,06 à 0,16 g (0,006 à 0,016 %) |
| - Ubiquinol : | au moins 0,06 g (0,006%), de préférence de 0,06 à 0,16 g (0,006 à 0,016 %) |
| - Acide lipoïque : | au moins 0,01 g (0,001%), de préférence de 0,01 à 0,015 g (0,001 à 0,0015 %) |

3. Milieu de culture selon l'une des revendications 1 ou 2, **caractérisé en ce qu'**il comprend en outre les composants nutritifs suivants dans le dit milieu de culture de base :
- plusieurs sources de carbone et d'azote, de préférence choisies parmi un extrait de levure, un sel d'acétate et une peptone trypsique,
- une source de phosphore, de préférence un sel de phosphate,
- au moins un sucre,
- au moins un sel de métaux choisis parmi K, Mg, Na et Ca, de préférence NaCl,
- ainsi que
- au moins une substance tampon régulateur de pH pour ajuster le pH de 7 à 8, de préférence K₂HPO₄, ou NaHCO₃ pour ajuster le pH à 7,5,
- au moins une mer vitamine ou facteur de croissance.

4. Milieu de culture selon l'une des revendications 1 à 3, **caractérisé en ce que** ledit milieu de culture de base est un milieu acellulaire conventionnel de bactérie comprenant des composants choisis parmi un extrait de broyat ou lysat de tissu pluricellulaire, un digestat enzymatique, notamment un digestat enzymatique de caséine, soja et/ou de tissu animal, une peptone, un extrait de levure, un sucre tel que dextrose ou glucose, un sel NaCl et/ou Na₂PO₄.

5. Milieu de culture selon l'une des revendications 1 à 4, **caractérisé en ce que** ledit milieu de culture de base est un milieu de culture liquide pour culture de bactéries anaérobies dans un échantillon sanguin, selle, crachat ou sécrétion vaginale.

6. Milieu de culture selon l'une des revendications 1 à 5, **caractérisé en ce que** ledit milieu de culture de base comprend les composants nutritifs suivants:
- Hydrolisat de caséine ;
- Protéose peptone ;
- Extrait de levure ;
- Glucose,
- Chlorure de sodium (NaCl) ; et
- un facteur de croissance : α-cétoglutarate.

7. Milieu de culture selon la revendication 6, **caractérisé en ce qu'**il comprend les composants suivants dans les quantités et proportions pondérales suivantes pour 1L :
| | |
|---|---|
| - Hydrolisat de caséine : | 15 g (1,5 %) |
| - Proteose peptone : | 15 g (1,5 %) |
| - Extrait de levure : | 10 g (1 %) |
| - α-cétoglutarate : | 2 g (0,2 %) |
| - Chlorure de sodium (NaCl) : | 5 g (0,5 %) |
| - Glucose: | 10 g (1 %) |
| - Dipotassium phosphate (K₂HPO₄) : | 0,83 g (0,083 %) |
| - Hydrosulfure de sodium (Na₂S) : | 0,5 g (0,05 %) |
| - L-Cystéine : | 0,5 g (0,05 %) |
| - Acide ascorbique : | 1 g (0,1 %) |
| - Glutathion : | 0,1 g (0,01 %) |
| - Catalase : | 0,16 g (0,016 %) |
| - Ubiquinol : | 0,16 g (0,016 %) |
| - Acide lipoïque : | 0,010 g (0,001 %) |
Le pH de la solution étant ajusté à 7,5±0,2 avec du KOH 10M.

8. Procédé de culture in vitro d'une bactérie aérobie ou anaérobie sous atmosphère aérobie avec un milieu de culture selon l'une des revendications 1 à 7.

9. Procédé de culture selon la revendication 8, **caractérisé en ce qu'**on réalise une culture d'une dite bactérie dans un échantillon de sang, selle, crachat ou sécrétion vaginale.

10. Procédé de culture selon la revendication 8 ou 9, **caractérisé en ce que** on réalise une culture d'une dite bactérie anaérobie stricte choisie parmi *Akkermansia muciniphila, Bacteroides fragilis, Bacteroides nordii, Bacteroides thetaiotaomicron, Clostridium beijeirinckii, Clostridium butyricum, Clostridium massilioamazoniensis, Clostridium irregulare, Finegoldia magna, Propionibacterium acnes et Propionibacterium avidum, Prevotella denticola et Prevotella histicola.*

## Patentansprüche

1. Polyvalentes flüssiges Kulturmedium für eine Kultur aus anaeroben Bakterien oder aeroben Bakterien in aerober Atmosphäre, umfassend ein Basiskulturmedium für aerobe und anaerobe Bakterien, **dadurch gekennzeichnet, dass** es ferner die Mischung aus folgenden Zusammensetzungen aus Antioxidantien aufweist: Natriumhydrosulfid (Na₂S), L-Cystein, Ascorbinsäure, Glutathion, Katalase, Ubiquinol und Liponsäure.

2. Kulturmedium nach Anspruch 1, **dadurch gekennzeichnet, dass** es die Zusammensetzungen aus Antioxidantien in folgenden Mengen und Gewichtsanteilen für 1 L umfasst:
| | |
|---|---|
| - Natriumhydrosulfid: | zumindest 0,25 g (0,025 %), bevorzugt 0,25 g bis 0,5 g (0,025 bis 0,05 %) |
| - L-Cystein: | zumindest 0,25 g (0,025 %), bevorzugt 0,25 g bis 0,5 g (0,025 bis 0,05 %) |
| - Ascorbinsäure: | zumindest 0,5 g (0,05 %), bevorzugt 0,5 g bis 1 g (0,05 bis 0,1 %) |
| - Glutathion: | zumindest 0,1 g (0,01 %), bevorzugt 0,1 g bis 0,5 g (0,01 bis 0,05 %) |
| - Katalase: | zumindest 0,06 g (0,006 %), bevorzugt 0,06 g bis 0,16 g (0,006 bis 0,016 %) |
| - Ubiquinol: | zumindest 0,06 g (0,006 %), bevorzugt 0,06 g bis 0,16 g (0,006 bis 0,016 %) |
| - Liponsäure: | zumindest 0,01 g (0,001 %), bevorzugt 0,01 g bis 0,015 g (0,001 bis 0,0015 %). |

3. Kulturmedium nach einem der Ansprüche 1 oder 2, **dadurch gekennzeichnet, dass** es ferner die folgenden Nährbestandteile in dem Basiskulturmedium umfasst:
- mehrere Kohlenstoff- und Stickstoffquellen, bevorzugt ausgewählt aus einem Hefeextrakt, einem Acetatsalz und einem tryptischen Pepton,
- eine Phosphorquelle, bevorzugt ein Phosphatsalz,
- zumindest einen Zucker,
- zumindest ein Salz von Metallen ausgewählt aus K, Mg, Na und Ca, bevorzugt NaCl,
- sowie
- zumindest eine pH-regulierende Puffersubstanz zum Einstellen des pH-Wertes von 7 auf 8, bevorzugt K₂HPO₄, oder NaHCO3 zum Einstellen des pH-Wertes auf 7,5,
- zumindest ein Vitamin oder einen Wachstumsfaktor.

4. Kulturmedium nach einem der Ansprüche 1 bis 3, **dadurch gekennzeichnet, dass** das Basiskulturmedium ein konventionelles zellfreies Bakterienmedium ist, das Bestandteile umfasst, die aus einem Extrakt aus Maische oder Lysat aus vielzelligem Gewebe, einem enzymatischen Gärrückstand, insbesondere einem enzymatischen Rückstand aus Kasein, Soja und/oder tierischem Gewebe, einem Pepton, einem Hefeextrakt, einem Zucker wie zum Beispiel Dextrose oder Glucose, einem NaCl- und/oder einem Na₂PO₄-Salz ausgewählt sind.

5. Kulturmedium nach einem der Ansprüche 1 bis 4, **dadurch gekennzeichnet, dass** das Basiskulturmedium ein flüssiges Kulturmedium für eine anaerobe Bakterienkultur in einer Blut-, Stuhl-, Speichel- oder Vaginalsekretprobe ist.

6. Kulturmedium nach einem der Ansprüche 1 bis 5, **dadurch gekennzeichnet, dass** das Basiskulturmedium die folgenden Nährbestandteile umfasst:
- Kaseinhydrolysat,
- Proteose-Pepton,
- Hefeextrakt,
- Glucose,
- Natriumchlorid (NaCl), und
- einen Wachstumsfaktor: α-Ketoglutarat.

7. Kulturmedium nach Anspruch 6, **dadurch gekennzeichnet, dass** es die folgenden Bestandteile in den folgenden Mengen und Gewichtsanteilen für 1 L umfasst:
| | |
|---|---|
| - Kaseinhydrolysat: | 15 g (1,5 %) |
| - Proteose-Pepton: | 15 g (1,5 %) |
| - Hefeextrakt: | 10 g (1 %) |
| - α-Ketoglutarat: | 2 g (0,2 %) |
| - Natriumchlorid (NaCl): | 5 g (0,5 %) |
| - Glucose: | 10 g (1 %) |
| - Dikaliumphosphat (K₂HPO₄): | 0,83 g (0,083 %) |
| - Natriumhydrosulfid (Na₂S): | 0,5 g (0,05 %) |
| - L-Cystein: | 0,5 g (0,05 %) |
| - Ascorbinsäure: | 1 g (0,1 %) |
| - Glutathion: | 0,1 g (0,01 %) |
| - Katalase: | 0,16 g (0,016 %) |
| - Ubiquinol: | 0,16 g (0,016 %) |
| - Liponsäure: | 0,010 g (0,001 %), |
wobei der pH-Wert der Lösung mit KOH 10M auf 7,5±0,2 eingestellt wird.

8. In-vitro-Kulturverfahren einer aeroben oder anaeroben Bakterie unter aerober Atmosphäre mit einem Kulturmedium nach einem der Ansprüche 1 bis 7.

9. Kulturverfahren nach Anspruch 8, **dadurch gekennzeichnet, dass** eine Kultur einer Bakterie in einer Blut-, Stuhl-, Speichel- oder Vaginalsekretprobe umgesetzt wird.

10. Kulturverfahren nach Anspruch 8 oder 9, **dadurch gekennzeichnet, dass** eine Kultur einer strengen anaeroben Bakterie umgesetzt wird, die aus *Akkermansia muciniphila, Bacteroides fragilis, Bacteroides nordii, Bacteroides thetaiotaomicron, Clostridium beijeirinckii, Clostridium butyricum, Clostridium massilioamazoniensis, Clostridium irregulare, Finegoldia magna, Propionibacterium acnes und Propionibacterium avidum, Prevotella denticola und Prevotella histicola* ausgewählt ist.

## Claims

1. A polyvalent liquid culture medium for culture in aerobic atmosphere of anaerobic bacteria or aerobic bacteria comprising a basal culture medium for aerobic and anaerobic bacteria **characterized in that** it further comprises the mixture of the following antioxidant compounds: sodium hydrosulphide (Na₂S), L-cysteine, ascorbic acid, glutathione, catalase, ubiquinol and lipoic acid.

2. The culture medium as claimed in claim 1, **characterized in that** it comprises said antioxidant compounds in the following quantities and weight proportions per 1 L:
| | |
|---|---|
| - Sodium hydrosulphide: | at least 0.25 g (0.025%), preferably from 0.25 g to 0.5 g (0.025 to 0.05%) |
| - L-Cysteine: | at least 0.25 g (0.025%), preferably from 0.25 g to 0.5 g (0.025 to 0.05%) |
| - Ascorbic acid: | at least 0.5 g (0.05%), preferably from 0.5 to 1 g (0.05 to 0.1%) |
| - Glutathione: | at least 0.1 g (0.01%), preferably from 0.1 to 0.5 g (0.01 to 0.05%) |
| - Catalase: | at least 0.06 g (0.006%), preferably 0.06 to 0.16 g (0.006 to 0.016%) |
| - Ubiquinol: | at least 0.06 g (0.006%), preferably 0.06 to 0.16 g (0.006 to 0.016%) |
| - Lipoic acid: | at least 0.01 g (0.001%), preferably from 0.01 to 0.015 g (0.001 to 0.0015%) |

3. The culture medium as claimed in one of claims 1 or 2, **characterized in that** it further comprises the following nutrient components in said basal culture medium:
- several sources of carbon and nitrogen, preferably selected from a yeast extract, an acetate salt and a tryptic peptone,
- a source of phosphorus, preferably a phosphate salt,
- at least one sugar,
- at least one salt of metals selected from K, Mg, Na and Ca, preferably NaCl,
- as well as
- at least one pH-regulating buffer substance for adjusting the pH from 7 to 8, preferably K₂HPO₄, or NaHCO3 for adjusting the pH to 7.5,
- at least one vitamin or growth factor.

4. The culture medium as claimed in one of claims 1 to 3, **characterized in that** said basal culture medium is a conventional acellular bacterial medium comprising components selected from an extract of ground material or lysate of multicellular tissue, an enzyme digestate, notably an enzyme digestate of casein, soybean and/or animal tissue, a peptone, a yeast extract, a sugar such as dextrose or glucose, a NaCl and/or Na₂PO₄ salt.

5. The culture medium as claimed in one of claims 1 to 4, **characterized in that** said basal culture medium is a liquid culture medium for culturing anaerobic bacteria in a blood, stool, sputum or vaginal secretion sample.

6. The culture medium as claimed in one of claims 1 to 5, **characterized in that** said basal culture medium comprises the following nutrient components:
- Casein hydrolysate;
- Proteose peptone;
- Yeast extract;
- Glucose,
- Sodium chloride (NaCI); and
- a growth factor: α-ketoglutarate.

7. The culture medium as claimed in claim 6, **characterized in that** it comprises the following components in the following quantities and weight proportions per 1 L:
| | |
|---|---|
| - Casein hydrolysate: | 15 g (1.5%) |
| - Proteose peptone: | 15 g (1.5%) |
| - Yeast extract: | 10 g (1%) |
| - α-Ketoglutarate: | 2 g (0.2%) |
| - Sodium chloride (NaCl): | 5 g (0.5%) |
| - Glucose: | 10 g (1%) |
| - Dipotassium phosphate (K₂HPO₄): | 0.83 g (0.083%) |
| - Sodium hydrosulphide (Na₂S): | 0.5 g (0.05%) |
| - L-Cysteine: | 0.5 g (0.05%) |
| - Ascorbic acid: | 1 g (0.1%) |
| - Glutathione: | 0.1 g (0.01%) |
| - Catalase: | 0.16 g (0.016%) |
| - Ubiquinol: | 0.16 g (0.016%) |
| - Lipoic acid: | 0.010 g (0.001%) |
The pH of the solution is adjusted to 7.5 ± 0.2 with 10 M KOH.

8. A process for the in vitro culture of an aerobic or anaerobic bacterium under aerobic atmosphere with a culture medium as claimed in one of claims 1 to 7.

9. The culture process as claimed in claim 8, **characterized in that** a said bacterium is cultured in a blood, stool, sputum or vaginal secretion sample.

10. The culture process as claimed in claim 8 or 9, **characterized in that** a said strict anaerobic bacterium selected from *Akkermansia muciniphila, Bacteroides fragilis, Bacteroides nordii, Bacteroides thetaiotaomicron, Clostridium beijeirinckii, Clostridium butyricum, Clostridium massilioamazoniensis, Clostridium irregulare, Finegoldia magna, Propionibacterium acnes* and *Propionibacterium avidum, Prevotella denticola* and *Prevotella histicola* is cultured.
